# EUROPEAN PATENT APPLICATION

(11) **EP 2 218 398 A1**
(43) Date of publication of application: **18.08.2010**
(21) Application number: 08761458.2
(22) Date of filing: 07.04.2008
(51) Int. Cl.: A61B 5/117, A61B 3/12, A61B 3/15, G06K 9/00

(54) **PERSON RECOGNITION METHOD AND DEVICE INCORPORATING THE ANATOMIC LOCATION OF THE RETINA AS A BIOMETRIC CONSTANT, CORRESPONDING TO THE PHYSIOLOGICAL LOCATION OF THE PROJECTION OF THE VISUAL AXIS**

(30) Priority: 27.11.2007 ES 200703141
(71) Applicant: UNIVERSIDAD COMPLUTENSE DE MADRID, 28040 Madrid (ES)
(72) Inventor: SANCHEZ RAMOS, Celia, E-28040 Madrid (ES); PANETSOS PETROVA, Fivos, E-28040 Madrid (ES)
(74) Representative: Temino Ceniceros, Ignacio
(86) International application number: PCT/ES2008/000211
(87) International publication number: WO 2009/068702

(57) **Abstract**

The invention relates to a person recognition method and device incorporating the anatomic location of the retina as a biometric constant, corresponding to the physiological location of the projection of the visual axis. More specifically, the invention relates to a person recognition method and a corresponding device incorporating the anatomic location of the retina as an exclusive discriminant biometric constant for each individual, corresponding to the physiological location of the visual axis, taking account of the physiological fixation disparity. The invention combines five elements, namely: an optical input system for examining the fundus of the eye, for example by means of retinal scanning or through the sclera; a system providing an expanded view of the retina, for example formed by converging lenses, diverging lenses and/or prisms or mirrors; a system for identifying and marking the anatomic point corresponding to the physiological point of the projectionof the visual axis, such as a laser; a system of filters having different transmittance and optical density values; and a system for capturing and processing digital images, such as an optical digital camera. In addition, the invention can take the form of a portable, auto-focusing device with automatic control.

## Description

### OBJECT OF THE INVENTION

The invention is part of the ophthalmology sector, applied to security and recognition of persons.

The purpose of this invention is a method and its corresponding device for the recognition of persons, which incorporates the anatomic location of the retina as an exclusive discriminating biometric constant for each individual, corresponding to the physiological location of the visual axis, which serves as an unmistakable reference point for the rest of the assessments, taking into account the physiological fixation disparity. This place is unique and invisible for each person as well as indeterminate without the proper device.

### STATE OF THE ART

The different types of biometric person recognition systems are based on the physical characteristics of the user to be identified. Although the identification of users via biometric methods is possible using any unique and measurable characteristic of the individual, it has traditionally been based on six large groups: Eye-iris, Eye-retina, fingerprints, geometry of the hand, writing-signature and voice.

Biometric authentication models based on ocular patterns are divided into two different technologies: they either analyze retina patterns or they analyze the morphological characteristics of the iris. Authentication via the retina can be currently carried out through the initial recording of the vascular structure of the retina (shape of the blood vessels of the human retina), which has characteristic elements of each individual and are different from the rest of the population.

In these systems, the user to be identified must look through an ocular device, adjust the distance and the movement of the head, look at a determined fixed point and, lastly, press a button to tell the device that he is ready for the analysis. In order to obtain valid records, you must wait five minutes for the retina mydriasis or dilation to occur, which is required in systems that enter through the pupil. Subsequently, the retina is scanned using low intensity infrared radiation; retina nodes and branches are detected in an image for comparing them with those stored in a database; if the sample coincides with that stored for the user the individual claims to be, the authentication is validated.

These methods are typically considered the most effective: for a population of millions of potential users since the probability of coincidence between individuals is practically non-existent. An additional characteristic of great importance in the authentication process is the fact that once the individual is dead, the ocular tissue degenerates rapidly, which makes it difficult for a false identification of intruders that can steal this organ from a corpse in order to falsify an authentication.

Other inventions related with this technique are known. Methods and devices for identifying persons based on retina characteristics have been described. For example, document US 4109237 describes a method and an identification system that entails scanning a persons eye for the purpose of detecting blood vessels that interfere with a determined light beam; document US 4877322 describes a method and a device that measures the relative oxygen saturation in the coroidal blood located at the bottom of a subject's eye when an incandescent red or infrared light source acts upon it; document US 2003091215 describes a biometric procedure based on the detection of personal trajectories of eye direction, including involuntary ocular movements when an image is observed.

Other inventions exist that refer more to methods and devices for taking the image of the retina or of a specific area of the retina. Thus, document WO 02075639 describes a system for taking the image of the retina by focusing the eye towards a determined point using two red and green light diodes; document WO 2006073781 refers to a system that detects if an image taken of the retina for use as a biometric constant is acceptable or if it needs to be repeated.

However, the use of these methods has certain disadvantages. Although the vascular pattern of the retina normally remains stable during one's entire life, it may be affected by diseases such as glaucoma, diabetes, high blood pressure or AIDS. On the other hand, methods based on the analysis of ocular patterns have little general acceptance since they use large size devices, which are difficult to handle. Additionally, these are very expensive systems for the majority of organizations and the authentication process is not as fast as it should be in situations where there are a large number of users. This way, their current use is practically limited to identification in high security systems such as the control of access to military installations.

The documents found in the state of the art differ from this invention in the fact that none of them considers the anatomical location of the retina, which corresponds, in the recording, to the physiological location of the projection of the visual axis.

The incorporation of the anatomical location of the retina that corresponds to the physiological location of the projection of the visual axis as a biometrical constant would provide important advantages regarding the current state of the art since it incorporates an infallible physiological constant: the point of view, without which the image could not be taken. Therefore:
- It completely cancels potential errors or deficiencies in measurement when taking the image.
- It is invisible and therefore cannot be reproduced without the proper device.
- Reduces the number of constants required for authentication.
- Facilitates and streamlines the required recording of the retina.
- Enables to reduce the required retina space and the size of the image and thus decreasing the costs.
- Enables to reduce the size of the device required for capturing and processing the image.

### DESCRIPTION OF THE INVENTION

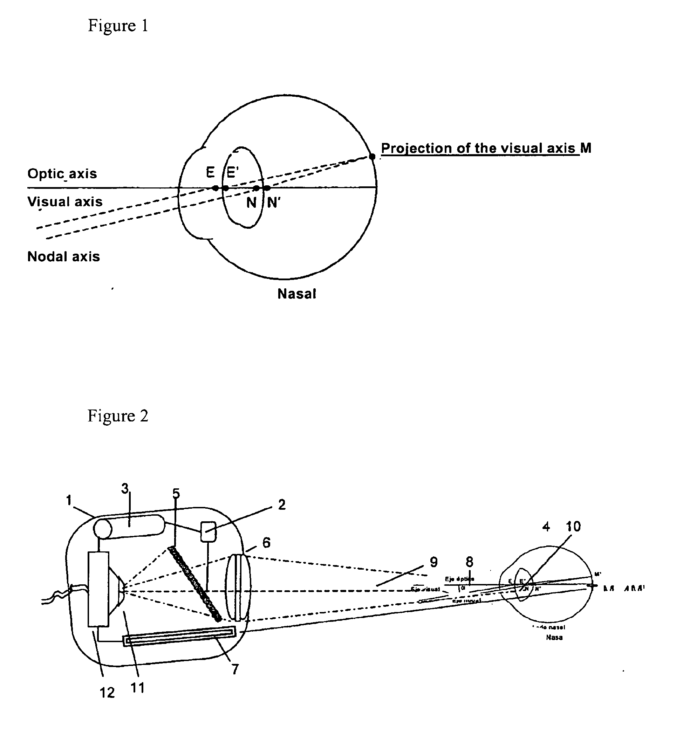

The purpose of the invention is a person recognition method and device incorporating the anatomic location of the retina as one of the biometric constants to be discriminated and as a point of reference the physiological location of the projection of the visual axis. This point, as shown in Figure 1, is normally located in the retina's fovea but the physiological fixation disparity must be taken into account.

Recognition of a person is accomplished by capturing an image of the retina, which among other constants includes the identification of that anatomic point and its recording and processing as data to be checked afterwards against the individual's identification records for validating or discarding the authentication.

The rest of the constants to be identified and validated may be, for example, the ordinate of the centre of the papilla, the abscissa of the centre of the papilla, the ordinate of the centre of the macula, the abscissa of the centre of the macula, the radius of the papilla, the ordinate and abscissa of the first artery bifurcation or upper temporal vein.

For this, the invention therefore incorporates a combination of five elements:
- An optical input system for examining the fundus of the eye, for example by means of retinal scanning or through the sclera.
- A transmittance and variable optic density filtering system.
- An expanded view of the retina system for example formed by converging lenses, diverging lenses and/or prisms or mirrors.
- A system for identifying and marking the anatomic point corresponding to the physiological point of the projection of the visual axis, like for example a laser, which must always be used as long as the individual looks at the point of fixation.
- A system for capturing and processing digital images such as an optical digital camera.

### DESCRIPTION OF THE FIGURES

Figure 1 is a schematic view of the position of the ocular axis, discriminating between visual axis (EV), Optic axis (EO) and nodal axis (EN), N the nodal point of the object, N' the nodal point of the image, E the optic centre of the object system, E' the optic centre of the image system and M' the projection of the visual axis.

Figure 2 represents the system purpose of this invention (1) that consists of a light emitting diode (2) that lights up the retina (4) where the visual axis is projected (10), a battery (3), a digital camera (12), a lens (11), an objective lens (6) that directs the reflected light from the retina through the mirror (5), an objective generator (7) for generating a view point that aligns the visual axis of an individual (8) with the central line (9).

### EMBODIMENT OF THE INVENTION

According to scientific literature and the current state of the art, several different ways exist for developing a method and a device of the same characteristics as the invention. Therefore, the embodiment method for this invention is illustrated in the following example, which is not limiting in scope since other ways and combinations of alternative devices exist.

Example of the embodiment of this invention as described in Figure 2: The system object of the patent (1) is comprised of a light emitting diode (2) connected to a battery (3) that illuminates the retina (4). The light is directed towards the retina using a mirror or prism (5) and a set of objective lenses (6). An objective generator (7), like for example a laser, generates a viewing point which when viewed by the individual, aligns the visual axis (8) with the central line (9). This way, the anatomical point that corresponds to the functional location of the visual axis projection (10) is illuminated by the light emitting diode (2) and identified by the objective generator (7). The objective lenses (6) collect and direct the light reflected from the retina via the mirror (5) to a lens (11) that is located on the front part of a digital photographic camera (12). The digital camera (12) is aligned with the central line of the lens (9) so the captured image represents the retina and the identification of the anatomic point corresponds to the location of the visual axis projection.

## Claims

1. Person recognition method based on the image of the retina, **characterized in that** it incorporates, as the biometric constant, the anatomic point corresponding to the physiological point of the visual axis projection, taking into account the fixation physiological disparity.

2. Person biometric recognition device based on the image of the retina comprised of (a) an optical input system for examining the retina through the pupil, via the sclera or other methods, (b) an expanded view of the retina system using converging lenses, diverging lenses and/or prisms, (c) a system that captures a digital image of the retina, (d) a system for identifying the anatomic point corresponding to the physiological point of the visual axis projection and (e) a system of filters of different transmittances and optic densities

3. A person recognition device in accordance with claim 2 that includes (a) a method that induces the eye to look at a fixation point and define the projection of its visual axis, (b) an optical input system for examining the retina through the pupil or the sclera (c) an expanded view of the retina system using converging lenses, diverging lenses and/or prisms, (d) a system that captures a digital image of the retina, (e) a system for identifying the anatomic point corresponding to the physiological point of the visual axis projection and (f) a system of filters of different levels of absorbance and optic densities, (g) a system for processing and output of an image in the form of data

4. A person recognition device according to claims 2 and 3 that may be portable.

5. A person recognition device according to claims 2 and 3 that may have auto focusing.

6. A person recognition device according to claims 2 and 3 that may have auto control.
